# FASCICULE DE BREVET EUROPEEN

(11) **EP 1 226 137 B1**
(45) Date de publication et mention de la délivrance du brevet: **21.07.2004**
(21) Numéro de dépôt: 00969646.9
(22) Date de dépôt: 19.10.2000
(51) Int. Cl.: C07D 401/06, A61K 31/4709, A61K 31/4725, A61P 9/00, C07D 401/14, C07D 217/02, C07D 215/14

(54) **PHENYL- ET PYRIDYL-TETRAHYDRO-PYRIDINES POSSEDANT UNE ACTIVITE INHIBITRICE DE FNT**
PHENYL- UND PYRIDYL-TETRAHYDRO-PYRIDINE MIT TNF INHIBITORISCHER WIRKUNG
PHENYL- AND PYRIDYL-TETRAHYDRO-PYRIDINES HAVING TNF INHIBITING ACTIVITY

(30) Priorité: 22.10.1999 FR 9913206; 28.06.2000 FR 0008328
(43) Date de publication de la demande: 31.07.2002
(73) Titulaire: SANOFI-SYNTHELABO, 75013 Paris (FR)
(72) Inventeur: BARONI, Marco, I-20010 Vanzago (IT); BOURRIE, Bernard, F-34980 Saint Gély du Fesc (FR); CARDAMONE, Rosanna, I-22100 Como (IT); CASELLAS, Pierre, F-34090 Montpellier (FR); GUZZI, Umberto, I-20148 Milano (IT)
(74) Mandataire: Varady, Peter
(86) Numéro de dépôt international: PCT/FR2000/002910
(87) Numéro de publication internationale: WO 2001/029026

(56) Documents cités:
- US-A- 3 991 061
- US-A- 5 118 691
- US-A- 5 776 939
- DATABASE CHEMABS [en ligne] CHEMICAL ABSTRACTS SERVICE, COLUMBUS, OHIO, US; BOURRIE, BERNARD ET AL: "The neuroprotective agent SR 57746A abrogates experimental autoimmune encephalomyelitis and impairs associated blood-brain barrier disruption: implications for multiple sclerosis treatment" retrieved from STN Database accession no. 132:30802 XP002142809 & PROC. NATL. ACAD. SCI. U. S. A. (1999), 96(22), 12855-12859,
- LOMBAERT S D ET AL: "Potent non-peptidic dual inhibitors of endothelin-converting enzyme and neutral endopeptidase 24.11" BIOORGANIC & MEDICINAL CHEMISTRY LETTERS,GB,OXFORD, vol. 7, no. 8, 22 avril 1997 (1997-04-22), pages 1059-1064, XP004136185 ISSN: 0960-894X

## Description

La présente invention concerne de nouvelles phényl- et pyridyl-tétrahydro-pyridines, les compositions pharmaceutiques les contenant, un procédé pour leur préparation et des intermédiaires de synthèse dans ce procédé.

US 5,118,691 et US 5,620,988 décrivent des tétrahydro-pyridmes, substituées par un radical quinolyl-3-alkyle, montrant une activité dopaminérgique.

Il a été maintenant trouvé que certaines tétrahydro-pyridines, substituées par un radical quinolinyl-alkyle ou isoquinolyl-alkyle, possèdent une puissante activité vis-à-vis de la modulation du TNF-alpha (de l'anglais Tumour Necrosis Factor).

Proc. Natl. Acad. Sci. U.S.A. (1999), 96 (22), 12855 - 12859 décrit un dérivé de 2-Naphthalényléthyl-Tétrahydropyridine et son utilisation comme inhihiteur du TNF-alpha.

Le TNF-alpha est une cytokine qui a récemment suscité de l'intérêt en tant que médiateur de l'immunité, de l'inflammation, de la prolifération cellulaire, de la fibrose etc. Ce médiateur est copieusement présent dans le tissu synovial enflammé et exerce un rôle important dans la pathogenèse de l'autoimmumté (Annu. Rep. Med. Chem., 1997, 32:241-250).

Ainsi, la présente invention concerne, selon un de ses aspects, des tétrahydro-pyridines de formule (I) : 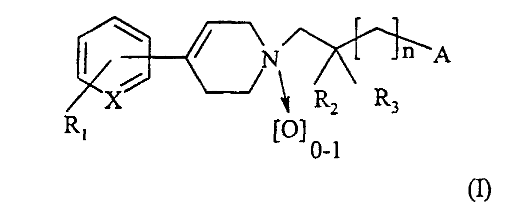 dans laquelle
- X: représente N ou CH;
- R₁: représente un atome d'hydrogène ou d'halogène ou un groupe CF₃ ;
- R₂ et R₃: représentent indépendamment un atome d'hydrogène ou un groupe méthyle ;
- n: est 0 ou 1 ;
- A: représente un groupe de formule (a) ou (b) 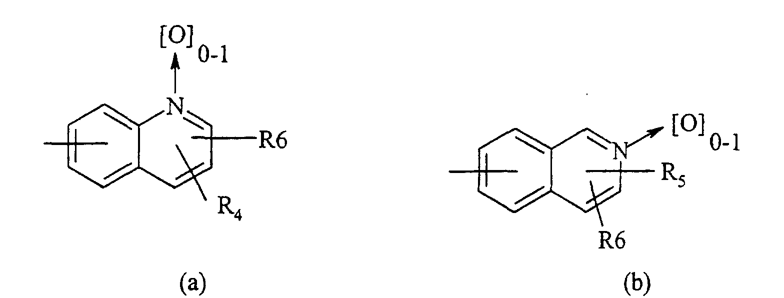
où
- R₄: représente un atome d'hydrogène ou d'halogène, un groupe (C₁-C₄) alkyle, un groupe CF₃, un groupe amino, mono(C₁-C₄)alkylamino, di(C₁-C₄)alkylamino ;
- R₅: représente un atome d'hydrogène ou d'halogène, un groupe (C₁-C₄)alkoxy, un groupe (C₁-C₄)alkyle ou un groupe CF₃ ;
- R₆: représente un atome d'hydrogène, un groupe (C₁-C₄)alkyle ou un groupe (C₁-C₄)alkoxy ;
ainsi que leurs sels ou solvates.

Dans la présente description, le terme "(C₁-C₄)alkyle" désigne un radical monovalent d'un hydrocarbure en C₁-C₄ saturé à chaîne droite ou ramifiée.

Dans la présente description, le terme "halogène" désigne un atome choisi parmi le chlore, le brome, le iode et le fluor.

Des composés préférés sont ceux où n est zéro.

D'autres composés préférés sont ceux où R₂ et R₃ sont l'hydrogène.

D'autres composés préférés sont ceux où R₁ est un groupe CF₃.

D'autres composés préférés sont ceux où R₁ est un atome de fluor.

D'autres composés préférés sont ceux où X est CH et R₁ est dans la position 2 ou 3 du benzène.

D'autres composés préférés sont ceux où X est CH et R₁ est un groupe CF₃.

D'autres composés préférés sont ceux où X est un atome d'azote et la pyridine est substituée dans les positions 2,6.

Selon la présente invention, les composés de formule (I) peuvent exister comme dérivés N-oxydes. Comme indiqué dans la formule ci-dessus, les composés de formule (I) peuvent notamment porter le groupe N-oxyde sur la tétrahydro-pyridine ou bien sur la quinoléine ou l'isoquinoléine du groupe A, ou encore deux groupes N-oxides peuvent simultanément être présents.

Les sels des composés de formule (I) selon la présente invention comprennent aussi bien les sels d'addition avec des acides minéraux ou organiques pharmaceutiquement acceptables tels que le chlorhydrate, le bromhydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I), tels que le picrate, l'oxalate ou les sels d'addition avec des acides optiquement actifs, par exemple les acides camphosulfoniques et les acides mandéliques ou mandéliques substitués.

Les stéréoisomères optiquement purs, ainsi que les mélanges d'isomères des composés de formule (I), dus au carbone asymétrique, quand l'un de R₂ et R₃ est un méthyle et l'autre un hydrogène, dans une proportion quelconque, font partie de la présente invention.

Les composés de formule (I) peuvent être synthétisés par un procédé qui prévoit
(a) de faire réagir le composé de formule (II): 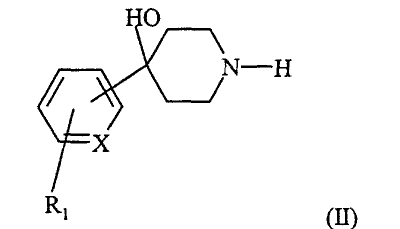 dans laquelle X et R₁ sont définis comme précédemment, avec un dérivé fonctionnel de l'acide de formule (III) : 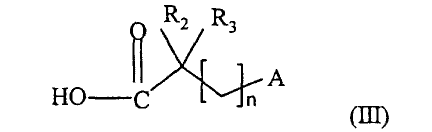 dans laquelle R₂, R₃, n et A sont tels que définis ci-dessus,
(b) de réduire le groupe carbonyle du composé de formule (IV) ainsi obtenu: 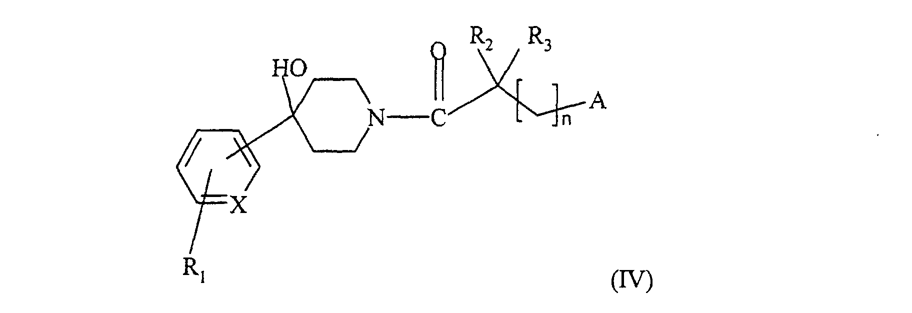
(c) de déshydrater le pipéridinol intermédiaire de formule (V) ainsi obtenu: 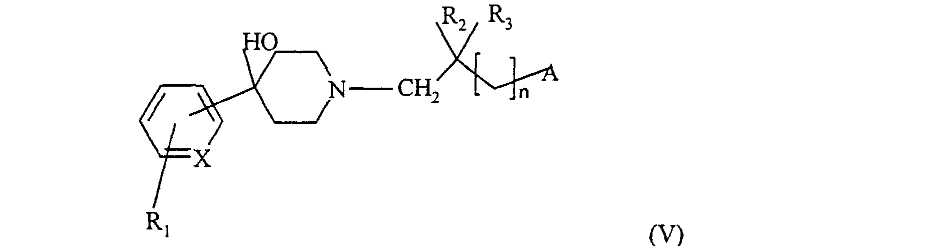
(d) d'isoler le composé de formule (I) ainsi obtenu et, éventuellement le transformer en l'un de ses sels ou solvates ou dans ses dérivés N-oxyde.

La réaction de l'étape (a) peut être convenablement conduite dans un solvant organique à une température comprise entre -10°C et la température de reflux du mélange réactionnel.

Il peut être préférable de réaliser la réaction à froid lorsqu'elle est exothermique, comme dans le cas où on utilise le chlorure en tant que dérivé fonctionnel de l'acide de formule (III).

Comme dérivé fonctionnel approprié de l'acide de formule (III), on peut utiliser l'acide libre, éventuellement activé (par exemple avec le BOP = tri(diméthylamino)benzotriazol-1-yloxyphosphonium hexafluorophosphate), un anhydride, un anhydride mixte, un ester actif ou un halogénure d'acide, le bromure de préférence. Parmi les esters actifs, l'ester de p-nitrophényle est particulièrement préféré, mais les esters de méthoxyphényle, de trityle, de benzhydryle et similaires sont également convenables.

Comme solvant de réaction, on utilise de préférence un solvant halogéné tel que le chlorure de méthylène, le dichloroéthane, le 1,1,1-trichloroéthane, le chloroforme et similaires, mais aussi d'autres solvants organiques compatibles avec les réactifs employés, par exemple le dioxane, le tétrahydrofurane ou un hydrocarbure tel que l'hexane, peuvent être également employés.

La réaction peut être convenablement conduite en présence d'un accepteur de protons, par exemple d'un carbonate alcalin ou d'une amine tertiaire telle que la triéthylamine.

La réduction de l'étape (b) peut être convenablement réalisée par des agents de réduction appropriés tels que les complexes du borane, par exemple le diméthylsulfure de borane ([CH₃]₂S-BH₃), les hydrures d'aluminium ou un hydrure complexe de lithium et d'aluminium dans un solvant organique inerte à une température comprise entre 0°C et la température de reflux du mélange réactionnel, selon les techniques usuelles.

Par "solvant organique inerte" on entend un solvant qui n'interfère pas avec la réaction. De tels solvants sont par exemple les éthers, tel que l'éther diéthylique, le tétrahydrofurane (THF), le dioxane ou le 1,2-diméthoxyéthane.

Selon un mode opérationnel préférentiel, on opère avec le diméthylsulfure de borane utilisé en excès par rapport au composé (II) de départ, à la température de reflux éventuellement sous atmosphère inerte. La réduction est normalement terminée après quelques heures

La déshydratation de l'étape (c) est aisément conduite par exemple en utilisant un mélange acide acétique/acide sulfurique, à une température comprise entre la température ambiante et la température de reflux du solvant utilisé.

Selon une méthode préférée la réaction de l'étape (c) est conduite dans un mélange acide acétique/acide sulfurique dans un rapport de 3/1 en volume, en chauffant à la température d'environ 100°C pendant 1-3 heures.

Le composé voulu est isolé selon les techniques conventionnelles sous forme de base libre ou d'un de ses sels. La base libre peut être transformée dans un de ses sels par simple salification dans un solvant organique tel qu'un alcool, de préférence l'éthanol ou l'isopropanol, un éther comme le 1,2-diméthoxyéthane, l'acétate d'éthyle, l'acétone ou un hydrocarbure comme l'hexane.

Le composé de formule (I) obtenu est isolé selon les techniques usuelles et éventuellement transformé en un de ses sels ou solvates ou dans ses dérivés N-oxyde.

Les composés de formule (I) peuvent également être préparés par une réaction de condensation/réduction à partir d'un composé de formule (VI) : 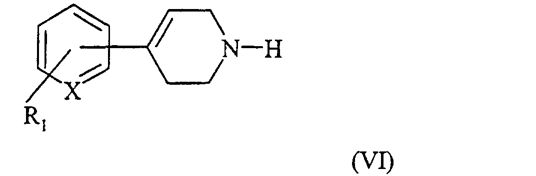 dans laquelle X et R₁ sont tels que définis ci-dessus, avec un aldéhyde de formule (VII) : 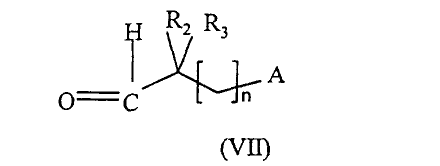 dans laquelle R₂, R₃, n et A sont tels que définis précédemment, isolement du composé de formule (I) et transformation éventuelle en un de ses sels ou solvates ou dans ses dérivés N-oxydes.

La réaction de condensation/réduction est conduite en mélangeant les composés de départ (VI) et (VII) dans un solvant organique tel qu'un alcool tel que par exemple le méthanol, en milieu acide, en présence d'un agent de réduction tel que le cyano-borohydrure de sodium, selon les méthodes conventionnelles.

Les composés de départ de formule (II), (III) et (VI) sont connus ou bien ils peuvent être préparés de façon analogue aux composés connus. De tels produits sont par exemple décrits dans WO 9701536 ; J.Am. Chem. Soc.,1948, 70:2843-2847 ; J. Med. Chem., 1997,40 (7):1049.

Les composés de formule (IV) et (V) sont des composé nouveaux et constituent un aspect ultérieur de la présente invention.

Les composé de formule (VII) peuvent être préparés en chauffant le trifluorométhylsulfonyl dérivé (autrement dit « triflate ») d'une hydroxy-(iso)quinoléine appropriée avec du N,N-dialkyléthanolamine vinyléther en présence d'un catalyseur de palladium et d'une base forte telle que par exemple la triéthylamine et en faisant réagir l'intermédiaire ainsi obtenu avec de l'acide sulfurique concentré, selon les procédures usuelles. Des exemples d'un tel procédé sont rapportés dans la partie expérimentale. Alternativement les composés de formule (VII) peuvent être préparés par réduction des correspondants acides de formule (IIII) selon les méthodes bien connues.

Les composés de formule (I) portant un groupe N-oxyde sur l'atome d'azote de la quinoléyne ou de l'isoquinoléyne peuvent être préparés à partir des dérivés N-oxyde des composés de formule (III) ou (VII). Des exemples de telles synthèses sont consignées dans la partie expérimentale.

Les composés de formule (I) portant un groupe N-oxyde sur l'atome d'azote de la tétrahydro-pyridine peuvent être préparés par oxydation des composé de formule (I) correspondant. Dans ce cas, le composé de formule (I) tel qu'obtenu par les synthèses ci-dessus, est soumis à une réaction d'oxydation selon les méthodes conventionnelles, par exemple à une réaction avec de l'acide m-chloro-perbenzoïque dans un solvant convenable et isolé selon les techniques usuelles bien connues à l'homme du métier.

Les composés de l'invention possèdent des propriétés intéressantes vis-à-vis de l'inhibition du TNF-α.

Ces propriétés ont été mises en évidence à l'aide d'un test visant à mesurer l'effet de molécules sur la synthèse du TNF-α induite chez la souris Balb/c par du lipopolysaccharide (LPS) d'Escherichia Coli (055 :B5, Sigma, St Louis, Mo).

Les produits à tester sont administrés par voie orale à des groupes de 5 souris Balb/c femelles (Charles River, France) âgées de 7 à 8 semaines. Une heure après, le LPS est administré par voie intravéneuse (10µg/souris). Le sang de chaque animal est prélevé 1,5 heure après l'administration du LPS. Les échantillons sont centrifugés, le plasma est récupéré et congelé à -80°C. Le TNF-α est mesuré à l'aide de kits commerciaux (R et D, Abingdon, UK).

Dans ce test, des composés représentatifs de l'invention se sont montrés très actifs, en inhibant la synthèse du TNF-α même à doses très faibles.

Grâce à cette activité et à leur faible toxicité, les composés de formule (I) et ses sels ou solvates peuvent bien être utilisés dans le traitement des maladies liées à des troubles immunitaires et inflammatoires ou comme analgésiques. Notamment les composés de formule (I) peuvent être utilisés pour traiter l'athérosclérose, les maladies autoimmunes, les maladies qui entraînent la démyélinisation des neurones (telles que la sclérose en plaques), l'asthme, l'arthrite rhumatoïde, les maladies fibrotiques, la fibrose idiopathique pulmonaire, la fibrose cystique, la glumérulonéphrite, la spondylite rhumatoïde, l'ostéoarthrite, la goutte, la résorbtion osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, le diabète, la cachexie, le cancer, les dommages liés aux radiations.

Les composés de formule (I) et leurs sels et solvates pharmaceutiquement acceptables sont de préférence administrés par voie orale.

Dans les compositions pharmaceutiques de la présente invention par voie orale, le principe actif peut être administré sous formes unitaires d'administration, en mélange avec des supports pharmaceutiques classiques, aux animaux et aux êtres humains pour le traitement des affections susmentionnées. Les formes unitaires d'administration appropriées comprennent par exemple les comprimés éventuellement sécables, les gélules, les poudres, les granules et les solutions ou suspensions orales.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

La quantité de principe actif à administrer dépend comme toujours du degré d'avancement de la maladie ainsi que de l'âge et du poids du patient. Néanmoins, les doses unitaires comprennent généralement de 0,001 à 100 mg, mieux de 0,01 à 50 mg, de préférence de 0,1 à 20 mg de principe actif, avantageusement de 0,5 à 10 mg.

Selon un autre de ses aspects, la présente invention concerne une association comprenant un composé de formule (I) ou l'un de ses sels ou solvates pharmaceutiquement acceptables, et au moins un composé choisi parmi les agents immunosuppresseurs, tel que l'interféron bêta-1b; l'hormone adrénocorticotrope; les glucocorticoïdes tels que la prednisone ou la méthylprednisolone; les inhibiteurs de l'interleukine-1.

Plus particulièrement, l'invention concerne une association comprenant un composé de formule (I), ou l'un de ses sels ou solvates pharmaceutiquement acceptables et au moins un composé choisi parmi le roquinimex (1,2-dihydro-4-hydroxy-N,1-diméthyl-2-oxo-3-quinolinecarboxanilide), le myloran (produit de la société Autoimmune contenant de la myéline bovine), l'antegren (anticorps humain monoclonal des sociétés Elan/Athena Neurosciences) l'interféron bêta-1b recombinant.

D'autres associations possibles sont celles constituées par un composé de formule (I) ou l'un de ses sels ou solvates pharmaceutiquement acceptables et un bloqueur des canaux potassiques, tel que par exemple la fampridine (4-aminopyridine).

Selon un autre de ses aspects, l'invention concerne une méthode de traitement des maladies liées à des troubles immunitaires et inflammatoires ainsi que dans le traitement de la douleur, notamment l'athérosclérose, les maladies autoimmunes, les maladies qui entraînent la démyélinisation des neurones (telles que la sclérose en plaques), l'asthme, l'arthrite rhumatoïde, les maladies fibrotiques, la fibrose idiopathique pulmonaire, la fibrose cystique, la glumérulonéphrite, la spondylite rhumatoïde, l'ostéoarthrite, la goutte, la résorption osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, l'attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, le diabète, la cachexie, le cancer, les dommages liés aux radiations, comprenant l'administration d'un composé de formule (I) ou de l'un de ses sels ou solvates pharmaceutiquement acceptables, seul ou en association avec d'autres principes actifs.

Les exemples qui suivent illustrent l'invention.

### PREPARATION 1

### 7-isoquinolyl-acétaldéhyde

On refroidit à 0°C 1,5 g (0,0103 mole) de 7-hydroxy-isoquinoléine et 5,3 ml de pyridine. On y ajoute goutte à goutte 1,86 ml d'anhydride triflique. On agite pendant 1 heure à 0°C et après 2 heures à la température ambiante. On verse dans un mélange eau/glace, on extrait à l'acétate d'éthyle, on sèche la phase organique et on évapore le solvant sous pression réduite. On purifie le brut par chromatographie sur colonne de gel de silice en éluant par un mélange cyclohexane/acétate d'éthyle = 7/3. On obtient la 7-hydroxy-isoquinoléine trifluorométhanesulfonate sous forme d'huile. On mélange sous argon 1,65 g de ce produit avec 27,5 ml de diméthylformamide anhydre, 41 mg de acétate de palladium, 1,65 ml de triéthylamine anhydre, 1,38 g de N,N-diéthyléthanolamine vinyléther. On chauffe à 80°C pendant 36 heures. On verse dans un mélange eau/acétate d'éthyle, on sépare les deux phases, on lave la phase organique à l'eau on sèche et on évapore le solvant sous pression réduite. On purifie le résidu par chromatographie sur colonne de gel de silice en éluant par un mélange acétate d'éthyle/méthanol = 9/1. On obtient la 2-[2-(7-isoquinolyl)éthenyl)oxy)]-N,N-diéthyl-1-éthanamine. On traite 1,5 g de ce produit avec 130 ml d'eau et 13 ml d'acide sulfurique à 96%. On chauffe pendant 4,5 heures à 60°C, on verse dans de la glace, on y ajoute une solution aqueuse saturée en NaHCO₃ et on extrait à l'acétate d'éthyle. On sèche la phase organique et on évapore le solvant sous pression réduite. On obtient le composé du titre.

### PREPARATION 2

### 6-isoquinolyl-acétaldéhyde.

En opérant comme décrit dans la préparation 1 mais en utilisant la 6-hydroxy-isoquinoléine on obtient le composé du titre.

### PREPARATION 3

### 7-isoquinolyl-acétaldéhyde-N-oxyde.

On mélange 14 ml d'eau, 3,5 ml d'acide sulfurique à 96% et 400 mg de 2-[2-(7-isoquinolyl)éthenyl)oxy)]-N,N-diéthyl-1-éthamine obtenue comme produit intermédiaire dans la Préparation 1. On y ajoute 24 ml de méthanol et on chauffe pendant 5,5 heures à 65°C. On verse dans de la glace, on y ajoute une solution aqueuse saturée en NaHCO₃ et on extrait à l'acétate d'éthyle. On sèche la phase organique et on évapore le solvant sous pression réduite. On purifie le produit par cromatographie sur colonne de gel de silice en éluant par un mélange cyclohexane/acétate d'éthyle=1/1 en obtenant la 7-(2,2-diméthoxyéthyl)isoquinoléyne. On dissout 100 mg de ce produit dans 15 ml de chlorure de méthylène et on y ajoute 135 mg d'acide m-chloro-perbenzonzoïque (MCPBA) et on agite pendant 3 heures à la température ambiante. On dilue avec du chlorure de méthylène, on ajoute une solution aqueuese de bicarbonate de sodium jusqu'à pH neutre, on extrait au chlorure de méthylène, on sèche sur du sulfate de sodium, on filtre et on évapore le solvant sous pression réduite en obtenant la 7-(2,2-diméthoxy-éthyl)isoquinoléyne-N-oxyde. On dissout 105 mg de ce produit dans 0,2 ml de chlorure de méthylène et on y ajoute à la température de 0°C 0,4 ml d'un mélange 1/1 d'acide trifluoro-acétique/eau. On agite à 0°C pendant 2 heures et après à la température ambiante pendant une nuit. On ajoute du chlorure de méthylène, on lave avec une solution de bicarbonate de sodium jusqu'à pH légèrement basique, on sèche la phase organique sur du sulfate de sodium, on filtre et on évapore le solvant sous pression réduite. On obtient le produit du titre.

### EXEMPLE 1

### 6-(2-(4-(3-trifluorométhyl-phényl)-1,2,3,6-tetrahydropyrid-1-yl)éthyl)quinoléine et son chlorhydrate.

### 1a) 1-(4-hydroxy-4-(3-trifluorométhyl-phényl)-1-pipéridinyl)-2-(6-quinoléinyl)-1-éthanone.

On agite pendant une nuit un mélange de 2,8 g (0,015 mole) d'acide 6-quinolylacétique, 4,2 g (0,0015 mole) de 4-hydroxy-4-(3-trifluorométhylphényl)pipéridine, 6,63 g (0,015 mole) de B.O.P, 5,3 ml de triéthylamine dans 60 ml de chlorure de méthylène. On ajoute 100 ml d'acétate d'éthyle, on lave à l'eau et avec une solution 1N d'hydroxyde de sodium, on sèche sur du sulfate de sodium et on évapore le solvant. On obtient 5,9 g du composé du titre.

### 1b) 6-(2-(4-hydroxy-4-(3-trifluométhyl-phényl)-1-pipéridinyl)éthyl) quinoleine.

On dissout dans 70 ml de THF anhydre le produit obtenu par l'exemple 1a, on chauffe au reflux et y on ajoute 4,05 ml (0,0427 mole) de diméthylsulfure de borane dans 50 ml de THF. On ajoute pendant 15 minutes à la température ambiante et après, 30 minutes au reflux. On évapore le solvant, on reprend le résidu dans le mélange acétate d'éthyle/NH₄OH dilué, on sépare les deux phases et on lave la phase organique à l'eau. On sèche sur du sulfate de sodium et on évapore le solvant sous pression réduite. On purifie le brut par chromatographie sur colonne de gel de silice en éluant par un mélange acétate d'éthyle/méthanol = 9/1. On obtient 1,95 g du produit du titre.
P.f. 140-142°C.

### 1c) 6-(2-(4-(3-trifluorométhyl-phényl)-1,2,3,6-tetrahydropyrid-1-yl)éthyl) quinoléine et son chlorhydrate.

On dissout 1,1 g du produit de l'étape précédente dans 12 ml d'acide acétique, on y ajoute 3,0 ml d'acide sulfurique concentré et on chauffe à 100°C pendant 2 heures. On verse dans du glaçon, on ajoute de l'NH₄OH dilué et on extrait à l'acétate d'éthyle. On lave à l'eau, on sèche et on évapore sous pression réduite. On purifie le brut par chromatographie en éluant par de l'acétate d'éthyle. On obtient le composé du titre. On prépare le chlorhydrate à l'aide d'une solution d'isopropanol saturé en acide chlorhydrique.
P.f. (chlorhydrate) 220-222°C.

### EXEMPLE 2

### 7-(2-(4-(3-trifluorométhyl-phényl)-1,2,3,6-tétrahydro-pyrid-1-yl)éthyl)quinoléine et son dichlorhydrate dihydraté.

En opérant comme décrit dans l'exemple 1 mais en utilisant l'acide 7-quinolylacétique au lieu de l'acide 6-quinolylacétique on obtient les composés du titre.
P.f. (dichlorhydrate dihydraté) 216-218°C.

### EXEMPLE 3

### 7-(2-(4-(3-trifluorométhyl-phényl)-1,2,3,6-tétrahydro-pyrid-1-yl)éthyl) isoquinoléine et son dichlorhydrate dihydraté.

On mélange 1,75 g (0,0077 mole) de 4-(3-trifluorométhyl-phényl)-1,2,3,6-tétrahydro-pyridine, 30 ml de méthanol, 1,15 ml d'acide acétique glaciale, 0,73 g d'acétate d'éthyle anhydre. On refroidit jusqu'à 0-5°C et on y ajoute 1,14 g de 7-isoquinolylacétataldéhyde (telle qu'obtenue par la Préparation 1) et avec précaution, 1,1 g (0,0175 mole) de cyanoborohydure de sodium. On agite pendant 1,5 heure à 0-5 °C et ensuite une nuit à la température ambiante. On ajoute 7 ml d'acide chlorhydrique concentré, on agite pendant 10 minutes, on évapore le solvant sous pression réduite, on reprend le résidu avec un mélange acétate d'éthyle/NH₄OH dilué. On sèche la phase organique sur du sulfate de sodium, on filtre et on évapore le solvant. On purifie le résidu sur colonne de gel de silice en éluant par un mélange acétate d'éthyle/méthanol = 9/1. On obtient le composé du titre. On prépare le chlorhydrate à l'aide d'une solution d'isopropanol saturée en acide chlorhydrique. On obtient 1,1 g de produit.
P.f. (dichlorhydrate dihydraté) 230-233°C.

### EXEMPLE 4

### 6-(2-(4-(3-trifluorométhyl-phényl)-1,2,3,6-tétrahydro-pyrid-1-yl)éthyl) isoquinoléine et son dichlorhydrate dihydraté.

En opérant comme décrit dans l'exemple 3 mais en utilisant la 6-isoquinolyl acétaldéhyde (telle qu'obtenue par la Préparation 2) au lieu de la 7-isoquinolyl acétaldéhyde on obtient les composés du titre.
P.f. (dichlorhydrate dihydraté) 222-224°C.

### EXEMPLE 5

### 6-(2-(4-(3-fluoro-phényl)-1,2,3,6-tétrahydro-pyrid-1-yl)éthyl)isoquinoléine et son dichlorhydrate.

En opérant comme décrit dans l'exemple 3 mais en utilisant la 6-isoquinolyl-acétaldéhyde (telle qu'obtenue par la Préparation 2) au lieu de la 7-isoquinolyl-acétaldéhyde et la 4-(3-fluoro-phényl)-1,2,3,6-tétrahydro-pyridine au lieu de la 4-(3-trifluorométhyl-phényl)-1,2,3,6-tétrahydro-pyridine, on obtient les composés du titre.
P.f. (dichlorhydrate) 242-244°C.

### EXEMPLE 6

### 7-(2-(4-(3-fluoro-phényl)-1,2,3,6-tétrahydro-pyrid-1-yl)éthyl)isoquinoléine et son dichlorhydrate.

En opérant comme décrit dans l'exemple 3 mais en utilisant la 4-(3-fluorophényl)-1,2,3,6-tétrahydro-pyridine au lieu de la 4-(3-trifluorométhyl-phényl)-1,2,3,6-tétrahydro-pyridine, on obtient les composés du titre.
P.f. (dichlorhydrate) 227-229°C.

### EXEMPLE 7

### 7-(2-(4-phényl-1,2,3,6-tétrahydro-pyrid-1-yl)éthyl)isoquinoléine et son dichlorhydrate.

En opérant comme décrit dans l'exemple 3 mais en utilisant la 4-phényl-1,2,3,6-tétrahydro-pyridine au lieu de la 4-(3-trifluorométhyl-phényl)-1,2,3,6-tétrahydro-pyridine, on obtient les composés du titre.
P.f. (dichlorhydrate) 259-261°C.

### EXEMPLE 8

### 6-(2-(4-(3-fluoro-phényl)-1,2,3,6-tétrahydro-pyrid-1-yl)éthyl)quinoléine et son dichlorhydrate.

En opérant comme décrit dans l'exemple 1 mais en utilisant la 4-hydroxy-4-(3-fluoro-phényl)pipéridine au lieu de la 4-hydroxy-4-(3-trifluorométhylphényl)pipéridine, on obtient les composés du titre.
P.f. (dichlorhydrate) 216-218°C.

### EXEMPLE 9

### 7-(2-(4-(3-trifluoromethyl-phényl)-1,2,3,6-tétrahydro-pyrid-1-oxyde-1-yl)éthyl) isoquinoléine et son chlorhydrate.

A une solution de 0,19 g (0,5 mmole) de 7-(2-(4-(3-trifluorométhyl-phényl)-1,2,3,6-tétrahydro-pyrid-1-yl)éthyl)isoquinoléine dans 25 ml de chlorure de méthylène à la température de 0-5°C on ajoute 0,086 g d'acide *m*-chloro-perbenzoïque. On laisse agiter à 0-5°C pendant deux heures , on lave à une solution aqueuse saturée en bicarbonate de sodium et on sépare les deux phases. On sèche la phase organique sur du sulfate de sodium on filtre et on évapore sous pression réduite. On purifie par flash chromatographie en éluant par un mélange méthanol/acétate d'éthyle=1/1 et on obtient le produit du titre. On prépare le chlorhydrate à l'aide d'une solution d'isopropanol saturée en acide chlorhydrique.
P.f. (chlorhydrate) 166-168°C.

### EXEMPLE 10

### 7-(2-(4-(3-trifluorométhyl-phényl)-1,2,3,6-tétrahydro-pyrid-1-yl)éthyl)isoquinoléine-N-oxyde et son chlorhydrate.

En opérant comme décrit dans l'exemple 3 mais en la 7-isoquinolyl-acétaldéhyde-N-oxyde (telle qu'obtenue par la Préparation 3) au lieu de la 7-isoquinolyl-acétaldéhyde on obtient les composés du titre.
P.f. (chlorhydrate) 198-201°C.

### EXEMPLE 11

### 7-(2-(4-(6-trifluorométhyl-pyrid-2-yl)-1,2,3,6-tétrahydro-pyrid-1-yl)éthyl) isoquinoléine et son dichlorhydrate dihydraté.

On mélange 0,650 g (0,0028 mole) de 4-(6-trifluorométhyl-pyrid-2-yl)-1,2,3,6-tétrahydro-pyridine, 16 ml de méthanol, 0,693 g d'acétate de sodium, 1,6 ml d'acide acétique et 1,05 g de 7-isoquinolyl acétaldéhyde (telle qu'obtenue par la Préparation 1) obtenu selon la préparation dans 16 ml de méthanol. On refroidit jusqu'à 0-5°C et, après 10 minutes, on y ajoute 1,06 g de cyanoborohydure de sodium, avec précaution. On agite pendant 30 minutes à 0-5 °C et ensuite une nuit à la température ambiante. On ajoute 7 ml d'acide chlorhydrique concentré, on agite pendant 30 minutes, on évapore le solvant sous pression réduite et on reprend le résidu avec un mélange éther diéthylique/NH₄OH dilué jusqu'à pH basique. On sèche la phase organique sur du sulfate de sodium, on filtre et on évapore le solvant. On purifie le résidu par flash-chromatographie sur colonne de gel de silice en éluant par un mélange acétate d'éthyle/méthanol = 9/1. On obtient le composé du titre (base) sous forme d'huile. On prépare le chlorhydrate à l'aide d'une solution d'isopropanol saturée en acide chlorhydrique. On obtient le composé du titre sous forme de son sel dichlorhydrate dihydraté.
P.f. (dichlorhydrate dihydraté) 203-206°C.

### EXEMPLE 12

### 6-(2-(4-(6-trifluorométhyl-pyrid-2-yl)-1,2,3,6-tétrahydro-pyrid-1-yl)éthyl) isoquinoléine et son dichlorhydrate.

En opérant comme décrit dans l'exemple 11, mais en utilisant la 6-isoquinolyl-acétaldéhyde au lieu de la 7-isoquinolyl-acétaldéhyde, on obtient les composés du titre.
P.f. (dichlorhydrate) 190-195°C.

### EXEMPLE 13

### 7-(2-(4-(6-chloro-pyrid-2-yl)-1,2,3,6-tétrahydro-pyrid-1-yl)éthyl)isoquinoléine et son dichlorhydrate.

En opérant comme décrit dans l'exemple 11, mais en utilisant la 4-(6-chloro-pyrid-2-yl)-1,2,3,6-tétrahydro-pyridine au lieu de la 4-(6-trifluorométhyl-pyrid-2-yl)-1,2,3,6-tétrahydro-pyridine, on obtient les composés du titre.
P.f. (dichlorhydrate) 110-112°C.

### EXEMPLE 14

### 7-(2-(4-(3-trifluorométhyl-phényl)-1,2,3,6-tétrahydro-pyrid-1-yl)éthyl)-1,3-diméthyl-isoquinoléine et son dichlorhydrate.

En opérant comme décrit dans l'exemple 3, mais en utilisant la 1,3-diméthyl-7-isoquinolylacétataldéhyde, on obtient les composés du titre.
P.f. (dichlorhydrate) 209°C.

### EXEMPLE 15

### 7-(2-(4-(3-trifluorométhyl-phényl)-1,2,3,6-tétrahydro-pyrid-1-yl)éthyl)-1,3-diéthyl-isoquinoléine et son dichlorhydrate.

En opérant comme décrit dans l'exemple 3, mais en utilisant la 1,3-diéthyl-7-isoquinolylacétataldéhyde, on obtient les composés du titre.
P.f. (dichlorhydrate) 192°C.

### EXEMPLE 16

### 7-(2-(4-(4-trifluorométhyl-phényl)-1,2,3,6-tétrahydro-pyrid-1-yl)éthyl) isoquinoléine et son dioxalate.

En opérant comme décrit dans l'exemple 3, mais en utilisant la 4-(4-trifluorométhyl-phényl)-1,2,3,6-tétrahydro-pyridine au lieu de la 4-(3-trifluorométhyl-phényl)-1,2,3,6-tétrahydro-pyridine, on obtient les composés du titre.
P.f. (dioxalate) 138-140°C.

### EXEMPLE 17

### 7-(2-(4-(2-trifluorométhyl-phényl)-1,2,3,6-tétrahydro-pyrid-1-yl)éthyl) isoquinoléine et son dichlorhydrate.

En opérant comme décrit dans l'exemple 3, mais en utilisant la 4-(2-trifluorométhyl-phényl)-1,2,3,6-tétrahydro-pyridine au lieu de la 4-(3-trifluorométhyl-phényl)-1,2,3,6-tétrahydro-pyridine, on obtient les composés du titre.
P.f. (dichlorhydrate) 158-160°C.

### EXEMPLE 18

### 7-(2-(4-(3-fluoro-phényl)-1,2,3,6-tétrahydro-pyrid-1-yl)éthyl) quinoléine et son chlorhydrate.

En opérant comme décrit dans l'exemple 1, mais en utilisant la 4-(3-fluorophényl)-1,2,3,6-tétrahydro-pyridine au lieu de la 4-(3-trifluorométhyl-phényl)-1,2,3,6-tétrahydro-pyridine et l'acide 7-quinolylacétique au lieu de l'acide 6-quinolylacétique, on obtient les composés du titre.
P.f. (chlorhydrate) 132°C.

### EXEMPLE 19

### 7-(2-(4-(6-trifluorométhyl-pyrid-2-yl)-1,2,3,6-tétrahydro-pyrid-1-yl)éthyl) quinoléine et son dichlorhydrate.

En opérant comme décrit dans l'exemple 3, mais en utilisant la 4-(6-trifluorométhyl-pyrid-2-yl)-1,2,3,6-tétrahydro-pyridine au lieu de la 4-(3-trifluorométhyl-phényl)-1,2,3,6-tétrahydro-pyridine et la 7-quinolylacétaldéhyde au lieu de la 7-isoquinolylacétaldéhyde, on obtient les composés du titre.
P.f. (dichlorhydrate) 135-136°C.

### EXEMPLE 20

### 7-(2-(4-(6-trifluorométhyl-pyrid-2-yl)-1,2,3,6-tétrahydro-pyrid-1-yl)éthyl) isoquinoléine N-oxyde

En opérant comme décrit dans l'exemple 3, mais en utilisant la 4-(6-trifluorométhyl-pyrid-2-yl)-1,2,3,6-tétrahydro-pyridine au lieu de la 4-(3-trifluorométhyl-phényl)-1,2,3,6-tétrahydro-pyridine et la 7-isoquinolyl-acétaldéhyde-N-oxyde (telle qu'obtenue par la Préparation 3) au lieu de la 7-isoquinolyl-acétaldéhyde, on obtient les composés du titre.

### EXEMPLES 21-26

En opérant comme décrit dans les exemples qui précèdent, on prépare les composés suivants :
la **7-(2-(4-(3-trifluorométhyl-phényl)-1,2,3,6-tétrahydro-pyrid-1-yl)éthyl)-3-méthyl-isoquinoléine ;**
la **7-(2-(4-(3-trifluorométhyl-phényl)-1,2,3,6-tétrahydro-pyrid-1-yl)éthyl)-1-méthyl-isoquinoléine ;**
la **7-(2-(4-(3-trifluorométhyl-phényl)-1,2,3,6-tétrahydro-pyrid-1-yl)éthyl)-3-éthyl-isoquinoléine. P.f. (dichlorhydrate) 230°C;**
la **7-(2-(4-(3-trifluorométhyl-phényl)-1,2,3,6-tétrahydro-pyrid-1-yl)éthyl)-1-éthyl-isoquinoléine ;**
la **7-(2-(4-(3-trifluorométhyl-phényl)-1,2,3,6-tétrahydro-pyrid-1-yl)éthyl)-3-méthoxy-isoquinoléine ;**
**la 7-(2-(4-(3-trifluorométhyl-phényl)-1,2,3,6-tétrahydro-pyrid-1-yl)éthyl)-2-méthyl-quinoléine. P.f. (dichlorhydrate) 221°C.**

## Revendications

1. Composé de formule (I) : 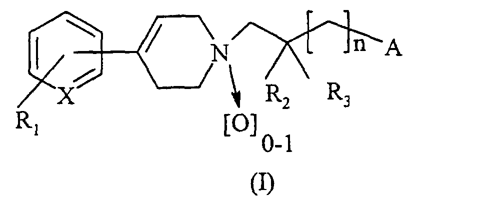 dans laquelle
X représente N ou CH ;
R₁ représente un atome d'hydrogène ou d'halogène ou un groupe CF₃ ;
R₂ et R₃ représentent indépendamment un atome d'hydrogène ou un groupe méthyle ;
n est 0 ou 1 ;
A représente un groupe de formule (a) ou (b) 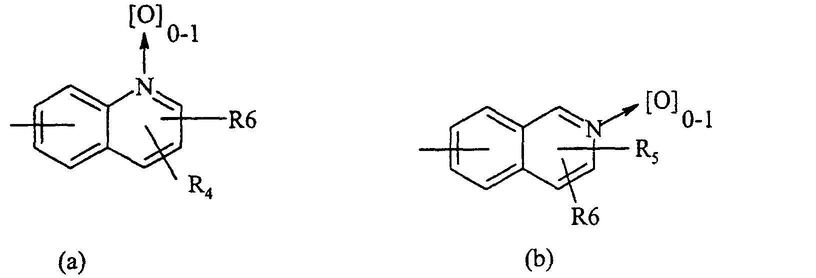
où
R₄ représente un atome d'hydrogène ou d'halogène, un groupe (C₁-C₄) alkyle, un groupe CF₃, un groupe amino, mono(C₁-C₄)alkylamino, di(C₁-C₄)alkylamino ;
R₅ représente un atome d'hydrogène ou d'halogène, un groupe (C₁-C₄)alkoxy, un groupe (C₁-C₄)alkyle ou un groupe CF₃ ;
R₆ représente un atome d'hydrogène, un groupe (C₁-C₄)alkyle ou un groupe (C₁-C₄)alkoxy ;
ainsi que ses sels ou solvates.

2. Composé selon la revendication 1 où n est zéro.

3. Composé selon les revendications 1 ou 2 où R₂ et R₃ sont l'hydrogène.

4. Composé selon les revendications de 1 à 3 où R₁ est un groupe CF₃.

5. Composé selon les revendications de 1 à 3 où R₁ est un atome de fluor.

6. Composé selon les revendications de 1 à 3 où X est CH et R₁ est dans la position 3 du benzène.

7. Composé selon les revendications de 1 à 3 où X est CH et R₁ est dans la position 2 du benzène.

8. Composé selon les revendications de 1 à 3 où X est un atome d'azote et la pyridine est substituée dans les positions 2,6.

9. Composé selon les revendications de 1 à 8 choisi parmi ses dérivés mono-N-oxyde et ses bis-N-oxyde.

10. Composé selon la revendication 1 choisi parmi la 7-(2-(4-(3-trifluorométhyl-phényl)-1,2,3,6-tétrahydro-pyrid-1-yl)éthyl) isoquinoléine, ses dérivés mono-N-oxyde, bis-N-oxyde, ses sels et solvates.

11. Procédé pour la préparation du composé de la revendication 1, **caractérisé en ce qu'**on effectue une réaction de condensation/réduction d'un composé de formule (VI) : 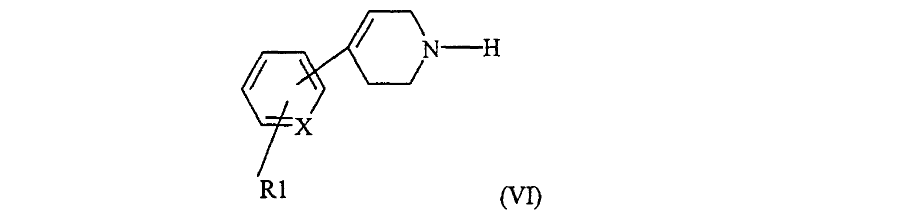 dans laquelle X et R₁ sont tels que définis dans la revendication 1, avec un aldéhyde de formule (VII) : 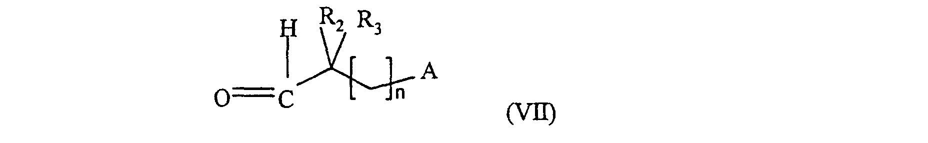 dans laquelle R₂, R₃, n et A sont tels que définis dans la revendication 1, l'isolement du composé de formule (I) et la transformation éventuelle en l'un de ses sels ou solvates ou ses N-oxydes.

12. Procédé pour la préparation du composé de la revendication 1, **caractérisé en ce que**
(a) on fait réagir le composé de formule (II) : 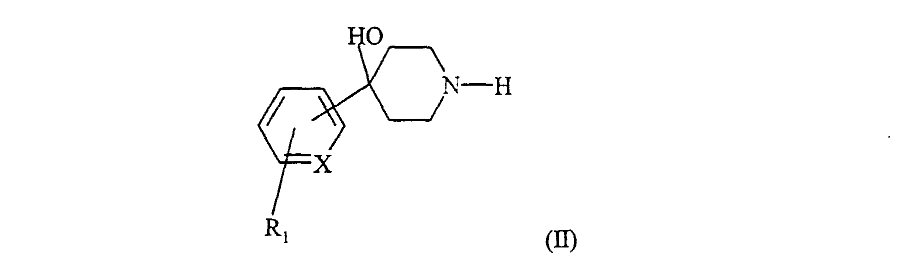 dans laquelle X et R₁ sont définis comme dans la revendication 1, avec un dérivé fonctionnel de l'acide de formule (III) : 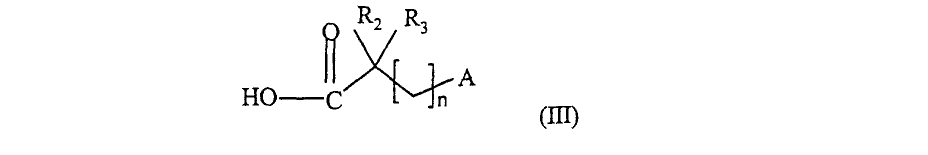 dans laquelle R₂, R₃, n et A sont tels que définis dans la revendication 1,
(b) on réduit le groupe carbonyle du composé de formule (IV) ainsi obtenu: 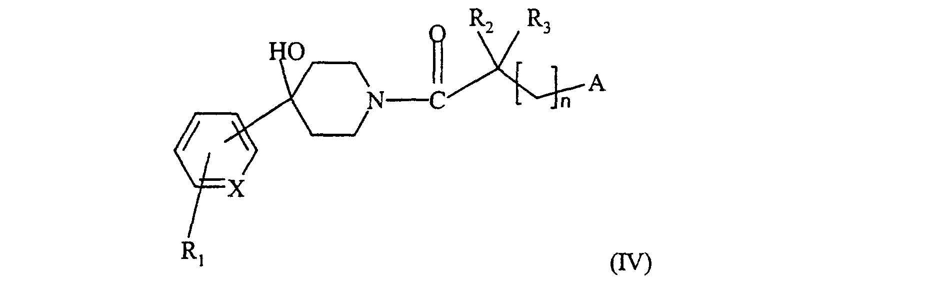
(c) on déshydrate le pipéridinol intermédiaire de formule (V) ainsi obtenu: 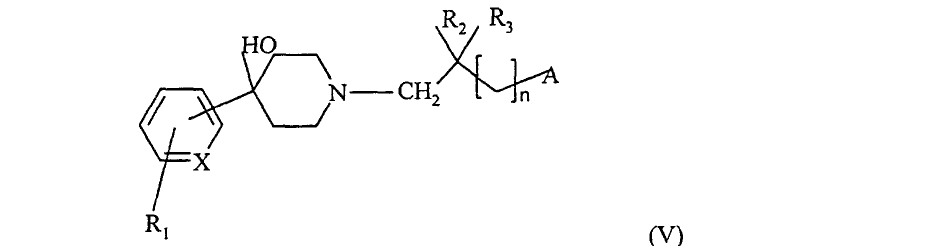
(d) On isole le composé de formule (I) ainsi obtenu et, éventuellement, on le transforme en l'un de ses sels ou solvates ou en ses dérivés N-oxyde.

13. Composé de formule (V) 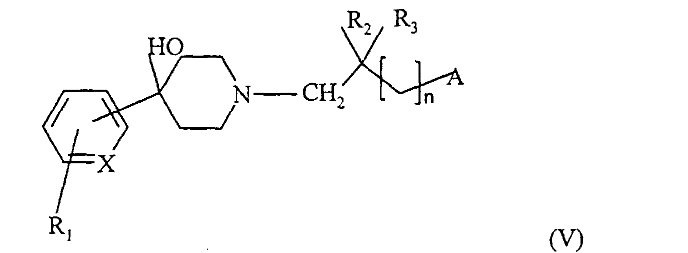 dans laquelle
x représente N ou CH;
R₁ représente un atome d'hydrogène ou d'halogène ou un groupe CF₃;
R₂ et R₃ représentent indépendamment un atome d'hydrogène ou un groupe méthyle ;
n est 0 ou 1 ;
A représente un groupe de formule (a) ou (b) 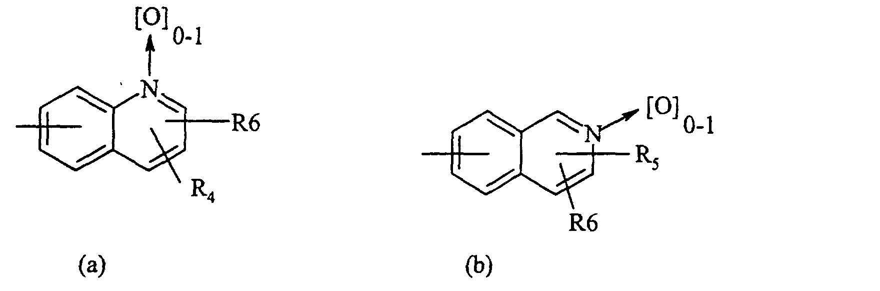
où
R₄ représente un atome d'hydrogène ou d'halogène, un groupe (C₁-C₄) alkyle, un groupe CF₃, un groupe amino, mono(C₁-C₄)alkylammo, di(C₁-C₄)alkylamino ;
R₅ représente un atome d'hydrogène ou d'halogène, un groupe (C₁-C₄)alkoxy, un groupe (C₁-C₄)alkyle ou un groupe CF₃ ;
R₆ représente un atome d'hydrogène, un groupe (C₁-C₄)alkyle ou un groupe (C₁-C₄)alkoxy ;
ainsi que ses sels ou solvates.

14. Composé de formule (IV) 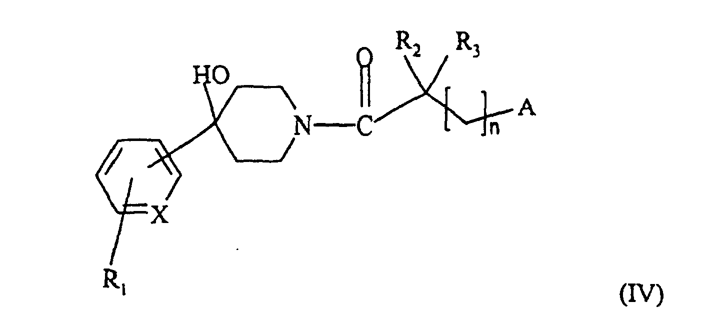 dans laquelle
X représente N ou CH ;
R₁ représente un atome d'hydrogène ou d'halogène ou un groupe CF₃;
R₂ et R₃ représentent indépendamment un atome d'hydrogène ou un groupe méthyle ;
n est 0 ou 1 ;
A représente un groupe de formule (a) ou (b) 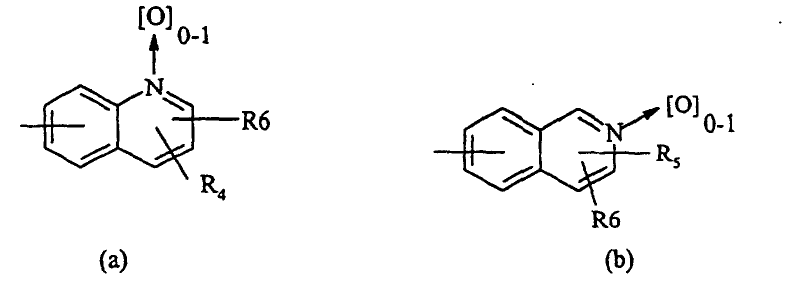
où
R₄ représente un atome d'hydrogène ou d'halogène, un groupe (C₁-C₄) alkyle, un groupe CF₃, un groupe amino, mono(C₁-C₄)alkylamino, di(C₁-C₄)alkylamino ;
R₅ représente un atome d'hydrogène ou d'halogène, un groupe (C₁-C₄)alkoxy, un groupe (C₁-C₄)alkyle ou un groupe CF₃;
R₆ représente un atome d'hydrogène, un groupe (C₁-C₄)alkyle ou un groupe (C₁-C₄)alkoxy ;
ainsi que ses sels ou solvates.

15. Composition pharmaceutique contenant en tant que principe actif un composé de formule (I) selon les revendications 1 à 10 ou un de ses sels ou solvates pharmaceutiquement acceptables.

16. Composition selon la revendication 15, **caractérisée en ce qu'**elle contient de 0,001 à 100 mg de principe actif.

17. Utilisation d'un composé de formule (I) selon les revendications 1 à 10 ou d'un de ses sels ou solvates pharmaceutiquement acceptables pour la préparation de médicaments analgésiques et/ou destinés au traitement des maladies liées à des troubles immunitaires et inflammatoires.

## Patentansprüche

1. Verbindung der Formel (I): 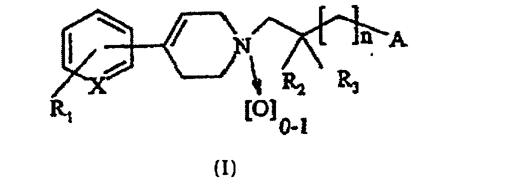 in der
X N oder CH bedeutet;
R₁ ein Wasserstoffatom, ein Halogenatom oder eine CF₃-Gruppe darstellt;
R₂ und R₃ unabhängig voneinander ein Wasserstoffatom oder eine Methylgruppe bedeuten;
n 0 oder 1 bedeutet;
A eine Gruppe der Formel (a) oder (b) darstellt 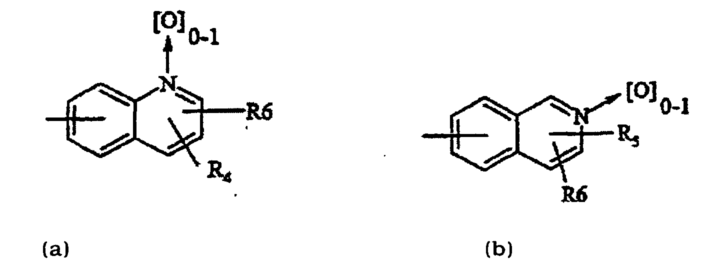
in denen
R₄ ein Wasserstoffatom, ein Halogenatom, eine (C₁-C₄)-Alkylgruppe, eine CF₃-Gruppe, eine Aminogruppe, eine Mono-(C₁-C₄)-alkylaminogruppe oder eine Di-(C₁-C₄)-alkylaminogruppe bedeutet;
R₅ ein Wasserstoffatom, ein Halogenatom, eine (C₁-C₄)-Alkoxygruppe, eine (C₁-C₄)-Alkylgruppe oder eine CF₃-Gruppe darstellt; und
R₆ ein Wasserstoffatom, eine (C₁-C₄)-Alkylgruppe oder eine (C₁-C₄)-Alkoxygruppe bedeutet;
sowie deren Salze oder Solvate.

2. Verbindung nach Anspruch 1, in der n Null bedeutet.

3. Verbindung nach den Ansprüchen 1 oder 2, worin R₂ und R₃ Wasserstoff bedeutet.

4. Verbindung nach den Ansprüchen 1 bis 3, worin R₃ eine CF₃-Gruppe darstellt.

5. Verbindung nach den Ansprüchen 1 bis 3, worin R₁ ein Fluoratom bedeutet.

6. Verbindung nach den Ansprüchen 1 bis 3, worin X CH bedeutet und R₁ in der 3-Stellung des Benzolkerns steht.

7. Verbindung nach den Ansprüchen 1 bis 3, worin X CH bedeutet und R₁ in der 2-Stellung des Benzolkerns steht.

8. Verbindung nach den Ansprüchen 1 bis 3, worin X ein Stickstoffatom bedeutet und der Pyridinrest in den Positionen 2,6 substituiert ist.

9. Verbindung nach den Ansprüchen 1 bis 8, ausgewählt aus seinen Mono-N-oxid- und seinen Bis-N-oxid-Derivaten.

10. Verbindung nach Anspruch 1, ausgewählt aus 7-(2-(4-(3-Trifluormethylphenyl)-1,2,3,6-tetrahydro-pyrid-1-yl)-ethyl)-isochinolin, dessen Mono-N-oxid- und Bis-N-oxid-Derivate, dessen Salze und Solvate.

11. Verfahren zur Herstellung der Verbindung nach Anspruch 1, **dadurch gekennzeichnet, daß** man eine Kondensations/Reduktions-Behandlung einer Verbindung der Formel (VI): 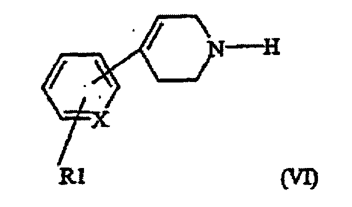 in der X und R₁ die in Anspruch 1 angegebenen Bedeutungen besitzen, mit einem Aldehyd der Formel (VII): 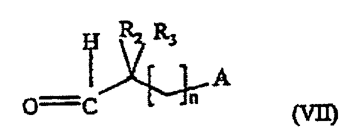 in der R₂, R₃, n und A die in Anspruch 1 angegebenen Bedeutungen besitzen, durchführt, die Verbindung der Formel (I) isoliert und gegebenenfalls in eines ihrer Salze oder Solvate oder N-Oxide umwandelt.

12. Verfahren zur Herstellung der Verbindung nach Anspruch 1, **dadurch gekennzeichnet, daß** man
(a) die Verbindung der Formel (II): 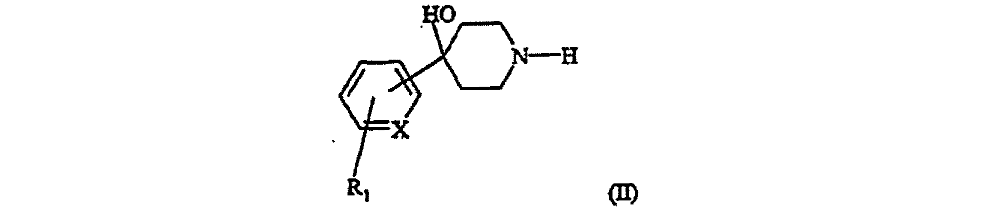 in der X und R₁ die in Anspruch 1 angegebenen Bedeutungen besitzen, mit einem funktionellen Derivat der Säure der Formel (III): 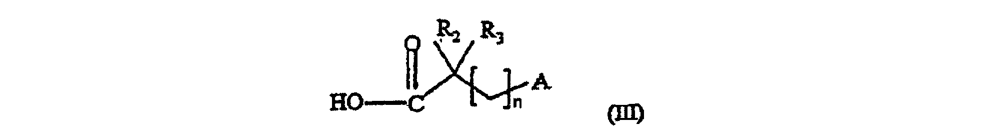 in der R₂, R₃, n und A die in Anspruch 1 angegebenen Bedeutungen besitzen, umsetzt.
(b) die Carbonylgruppe der in dieser Weise erhaltenen Verbindung der Formel (IV) reduziert: 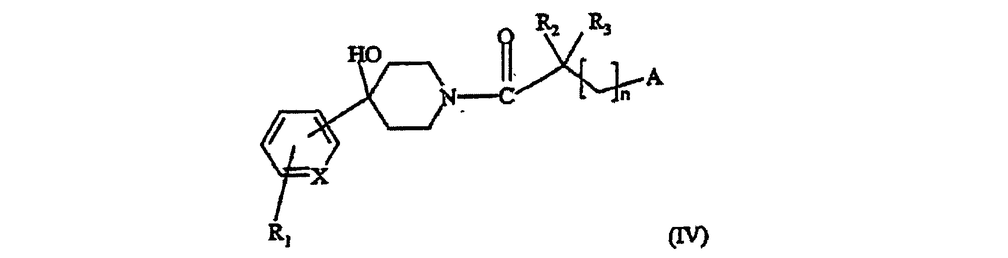
(c) das in dieser Weise als Zwischenprodukt der Formel (V) erhaltene Piperidinol deshydratisiert: 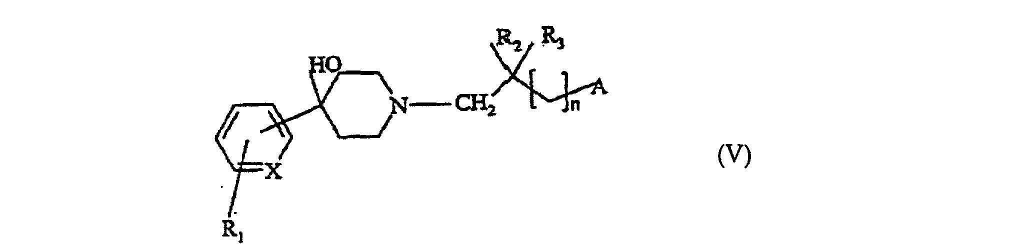
(d) die in dieser Weise erhaltene Verbindung der Formel (I) isoliert und sie gegebenenfalls in eines ihrer Salze oder Solvate oder N-Oxid-Derivate umwandelt.

13. Verbindung der Formel (V) 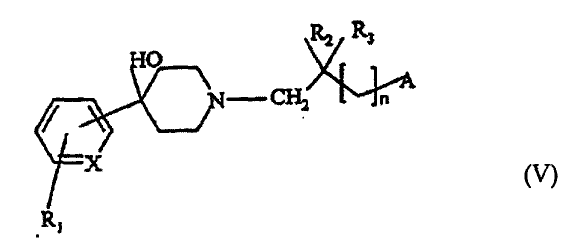 in der
X N oder CH bedeutet;
R₁ ein Wasserstoffatom, ein Halogenatom oder eine CF₃-Gruppe darstellt;
R₂ und R₃ unabhängig voneinander ein Wasserstoffatom oder eine Methylgruppe bedeuten;
n 0 oder 1 bedeutet;
A eine Gruppe der Formel (a) oder (b) darstellt 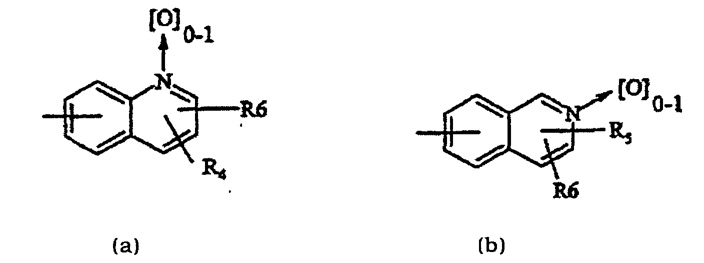
in denen
R₄ ein Wasserstoffatom, ein Halogenatom, eine (C₁-C₄)-Alkylgruppe, eine CF₃-Gruppe, eine Aminogruppe, eine Mono-(C₁-C₄)-alkylaminogruppe oder eine Di-(C₁-C₄)-alkylaminogruppe bedeutet;
R₅ ein Wasserstoffatom, ein Halogenatom, eine (C₁-C₄)-Alkoxygruppe, eine (C₁-C₄)-Alkylgruppe oder eine CF₃-Gruppe darstellt; und
R₆ ein Wasserstoffatom, eine (C₁-C₄)-Alkylgruppe oder eine (C₁-C₄)-Alkoxygruppe bedeutet;
sowie deren Salze oder Solvate.

14. Verbindung der Formel (IV) 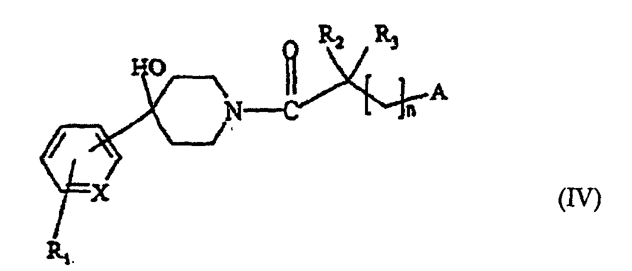 in der
X N oder CH bedeutet;
R₁ ein Wasserstoffatom, ein Halogenatom oder eine CF₃-Gruppe darstellt;
R₂ und R₃ unabhängig voneinander ein Wasserstoffatom oder eine Methylgruppe bedeuten;
n 0 oder 1 bedeutet;
A eine Gruppe der Formel (a) oder (b) darstellt 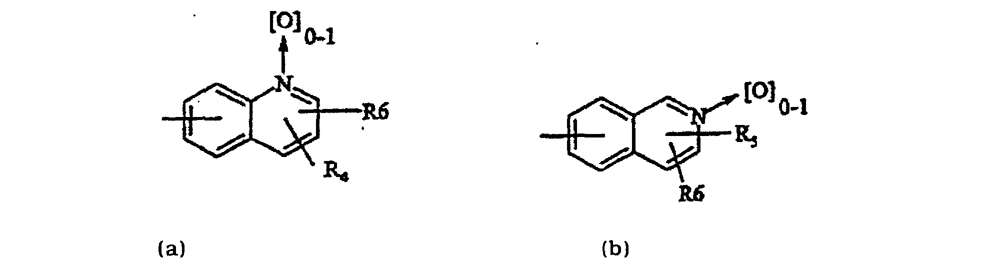
in denen
R₄ ein Wasserstoffatom, ein Halogenatom, eine (C₁-C₄)-Alkylgruppe, eine CF₃-Gruppe, eine Aminogruppe, eine Mono-(C₁-C₄)-alkylaminogruppe oder eine Di-(C₁-C₄)-alkylaminogruppe bedeutet;
R₅ ein Wasserstoffatom, ein Halogenatom, eine (C₁-C₄)-Alkoxygruppe, eine (C₁-C₄)-Alkylgruppe oder eine CF₃-Gruppe darstellt; und
R₆ ein Wasserstoffatom, eine (C₁-C₄)-Alkylgruppe oder eine (C₁-C₄)-Alkoxygruppe bedeutet;
sowie deren Salze oder Solvate.

15. Pharmazeutische Zubereitung enthaltend als Wirkstoff eine Verbindung der Formel (I) nach den Ansprüchen 1 bis 10 oder eines ihrer pharmazeutisch annehmbaren Salze oder Solvate.

16. Zubereitung nach Anspruch 15, **dadurch gekennzeichnet, daß** sie 0,001 bis 100 mg des Wirkstoffs enthält.

17. Verwendung einer Verbindung der Formel (I) nach den Ansprüchen 1 bis 10 oder eines ihrer pharmazeutisch annehmbaren Salze oder Solvate für die Herstellung von analgetischen Arzneimitteln und/oder solchen, die zur Behandlung von Krankheiten bestimmt sind, die mit Immunstörungen und Entzündungszuständen verknüpft sind.

## Claims

1. Compound of formula (I): 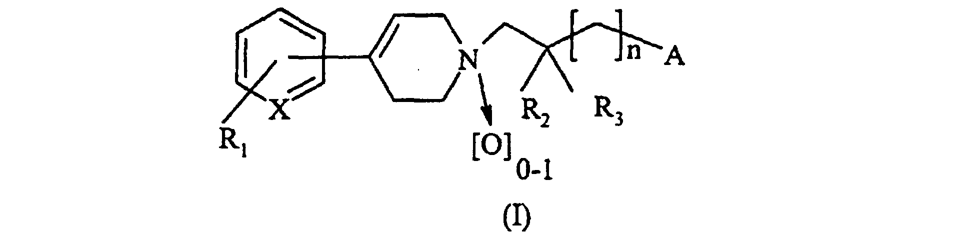 in which
X represents N or CH;
R₁ represents a hydrogen or halogen atom or a CF₃ group;
R₂ and R₃ , independently represent a hydrogen atom or a methyl group;
n is 0 or 1;
A represents a group of formula (a) or (b) 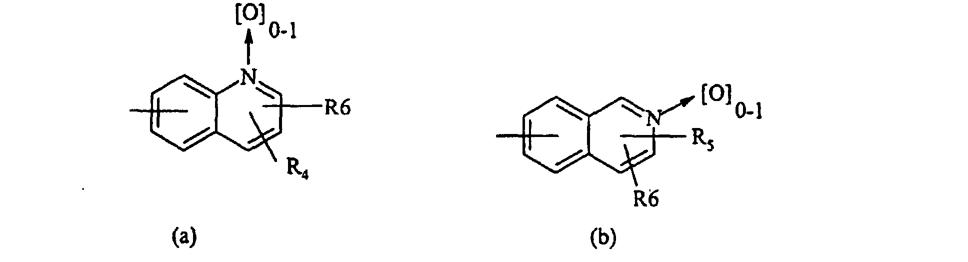
in which
R₄ represents a hydrogen or halogen atom, a (C₁-C₄)alkyl group, a CF₃ group, an amino group, a mono(C₁-C₄)alkylamino group or a di(C₁-C₄)alkylamino group;
R₅ represents a hydrogen or halogen atom, a (C₁-C₄)alkoxy group, a (C₁-C₄)alkyl group or a CF₃ group;
R₆ represents a hydrogen atom, a (C₁-C₄)alkyl group or a (C₁-C₄)alkoxy group;
as well as the salts or solvates thereof.

2. Compound according to Claim 1, in which n is zero.

3. Compound according to Claim 1 or 2, in which R₂ and R₃ are hydrogen.

4. Compound according to Claims 1 to 3, in which R₁ is a CF₃ group.

5. Compound according to Claims 1 to 3, in which R₁ is a fluorine atom.

6. Compound according to Claims 1 to 3, in which X is CH and R₁ is in position 3 of the benzene.

7. Compound according to Claims 1 to 3, in which X is CH and R₁ is in position 2 of the benzene.

8. Compound according to Claims 1 to 3, in which X is a nitrogen atom and the pyridine is substituted in positions 2 and 6.

9. Compound according to Claims 1 to 8, chosen from the mono-N-oxide and bis-N-oxide derivatives thereof.

10. Compound according to Claim 1, chosen from 7-(2-(4-(3-trifluoromethylphenyl)-1,2,3,6-tetrahydro-1-pyridyl)ethyl)isoquinoline, the mono-N-oxide and bis-N-oxide derivatives thereof and the salts and solvates thereof.

11. Process for preparing the compound of Claim 1, **characterized in that** a coupling/reduction reaction is carried out on a compound of formula (VI): 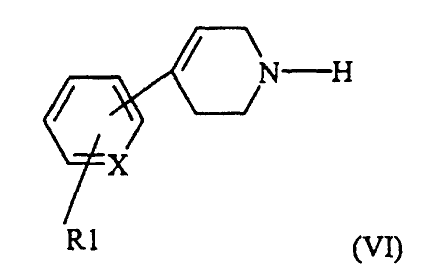 in which X and R₁ are as defined in Claim 1, with an aldehyde of formula (VII): 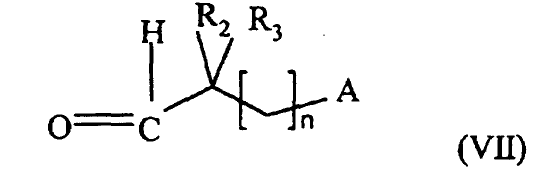 in which R₂, R₃, n and A are as defined in Claim 1, and the compound of formula (I) is isolated and optionally converted into a salt or solvate thereof or an N-oxide thereof.

12. Process for preparing the compound of Claim 1, **characterized in that**
(a) the compound of formula (II): 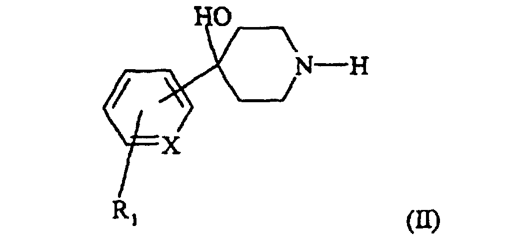 in which X and R₁ are defined as in Claim 1, is reacted with a functional derivative of the acid of formula (III): 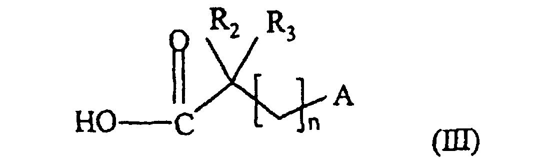 in which R₂, R₃, n and A are as defined in Claim 1,
(b) the carbonyl group of the compound of formula (IV) thus obtained: 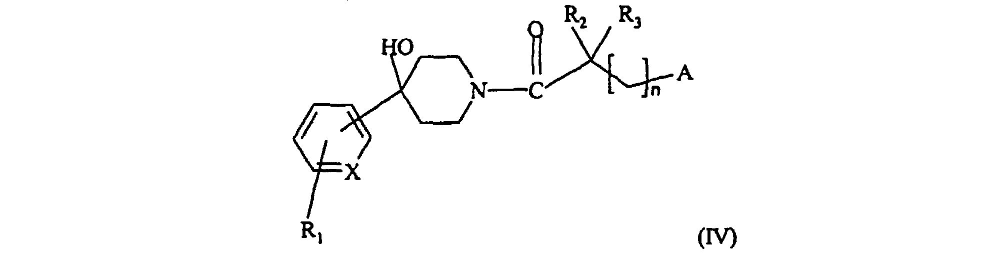 is reduced,
(c) the intermediate piperidinol of formula (V) thus obtained: 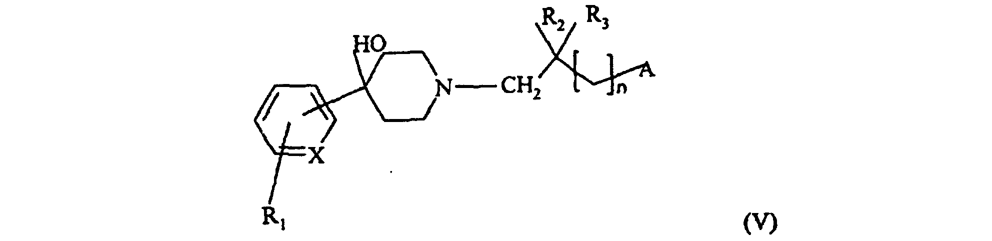 is dehydrated,
(d) the compound of formula (I) thus obtained is isolated and is optionally converted into a salt or solvate thereof or into the N-oxide derivatives thereof.

13. Compound of formula (V) 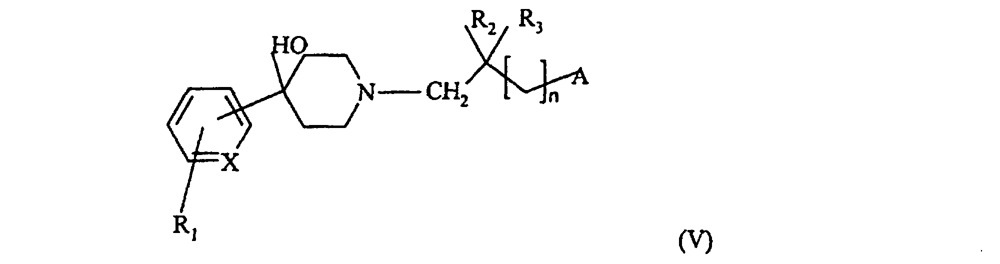 in which
X represents N or CH;
R₁ represents a hydrogen or halogen atom or a CF₃ group;
R₂ and R₃ independently represent a hydrogen atom or a methyl group;
n is 0 or 1;
A represents a group of formula (a) or (b) 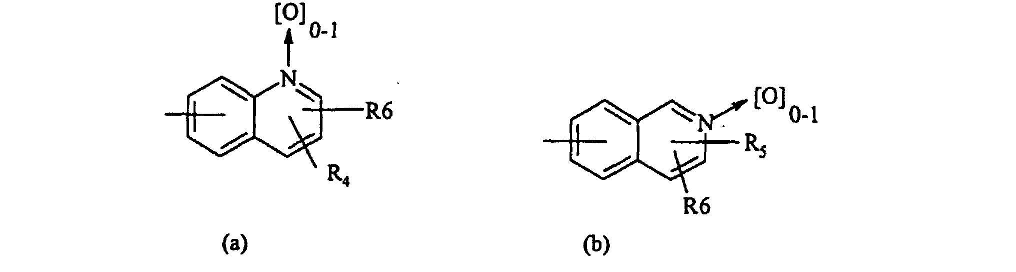
in which
R₄ represents a hydrogen or halogen atom, a (C₁-C₄)alkyl group, a CF₃ group, an amino group, a mono(C₁-C₄) alkylamino group or a di (C₁-C₄)alkylamino group;
R₅ represents a hydrogen or halogen atom, a (C₁-C₄)alkoxy group, a (C₁-C₄)alkyl group or a CF₃ group;
R₆ represents a hydrogen atom, a (C₁-C₄)alkyl group or a (C₁-C₄)alkoxy group;
as well as the salts or solvates thereof.

14. Compound of formula (IV) 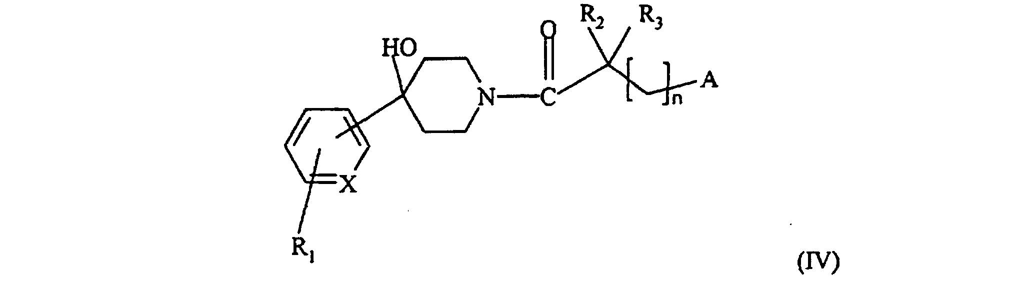 in which
X represents N or CH;
R₁ represents a hydrogen or halogen atom or a CF₃ group;
R₂ and R₃ independently represent a hydrogen atom or a methyl group;
n is 0 or 1;
A represents a group of formula (a) or (b) 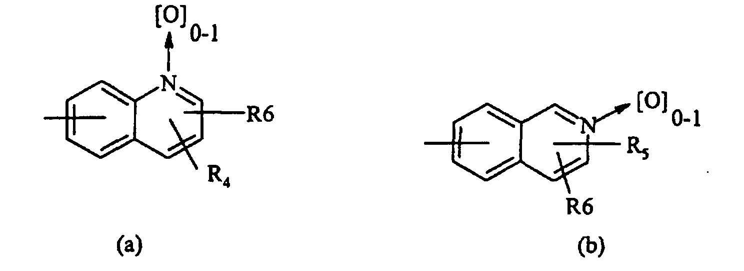
in which
R₄ represents a hydrogen or halogen atom, a (C₁-C₄)alkyl group, a CF₃ group, an amino group, a mono(C₁-C₄) alkylamino group or a di (C₁-C₄)alkylamino group;
R₅ represents a hydrogen or halogen atom, a (C₁-C₄)alkoxy group, a (C₁-C₄)alkyl group or a CF₃ group;
R₆ represents a hydrogen atom, a (C₁-C₄)alkyl group or a (C₁-C₄)alkoxy group;
as well as the salts or solvates thereof.

15. Pharmaceutical composition containing, as active principle, a compound of formula (I) according to Claims 1 to 10 or a pharmaceutically acceptable salt or solvate thereof.

16. Composition according to Claim 15, **characterized in that** it contains from 0.001 mg to 100 mg of active principle.

17. Use of a compound of formula (I) according to Claims 1 to 10, or of a pharmaceutically acceptable salt or solvate thereof, for the preparation of analgesic medicinal products, and/or medicinal products which are intended for the treatment of diseases associated with immune and inflammatory disorders.
